# EUROPEAN PATENT APPLICATION

(11) **EP 4 592 675 A1**
(43) Date of publication of application: **30.07.2025**
(21) Application number: 23899713.4
(22) Date of filing: 14.11.2023
(51) Int. Cl.: G01N 33/49, G01N 33/53

(54) **HYDROPHOBIC DISRUPTOR, PREPARATION METHOD THEREFOR, AND USE THEREOF**

(30) Priority: 06.12.2022 CN 202211555080
(71) Applicant: Shenzhen Yhlo Biotech Co., Ltd., Shenzhen, Guangdong 518116 (CN)
(72) Inventor: YANG, Yonghong, Shenzhen, Guangdong 518116 (CN); CHENG, Fangming, Shenzhen, Guangdong 518116 (CN); WANG, Gang, Shenzhen, Guangdong 518116 (CN); QIAN, Chungen, Shenzhen, Guangdong 518116 (CN)
(74) Representative: V.O.
(86) International application number: PCT/CN2023/131575
(87) International publication number: WO 2024/120133

(57) **Abstract**

A hydrophobic disruptor, a preparation method therefor, and a use thereof. The preparation method for the hydrophobic disruptor comprises the following steps: adding a lysing agent into a whole blood sample to fully lyse blood cells in the whole blood sample and release the contents, and centrifuging to extract a supernatant; and adding an oxidizing agent to the supernatant to oxidize the contents to improve hydrophobicity, centrifuging to extract a supernatant, and obtaining a hydrophobic disruptor. The prepared hydrophobic disruptor can be used in immunodiagnostic reagents such as POCT, ELISA, and CLIA to test the anti-interference performance against the hydrophobic disruptor. In clinical testing, potential interferences can be identified and optimized in advance to improve reagents and prevent false positive or false positive results generated in clinical sample testing.

## Description

### TECHNICAL FIELD

The present application relates to the field of in vitro diagnostic technology, and particularly to a hydrophobic interferent, and its preparation and use.

### BACKGROUND

Common interferences in an immunoreaction include endogenous and exogenous interferences. Common endogenous interferences include those by hemolysis, lipemia, total protein, analogs, elevated IgG or IgM, rheumatoid factor, heterophilic antibodies, biotin, etc. Common exogenous interferences include drug interference, bacterial or viral infections, etc. One fundamental cause of interferences in an immunoreaction is hydrophobic interaction, where the interferent non-specifically bridges a coating conjugate and a label conjugate, or bridges a reaction cup (reaction well) with a label conjugate via hydrophobic force, leading to a false-positive signal. Proteins are composed of amino acids, and different amino acids exhibit varying degrees of hydrophilicity and hydrophobicity. Consequently, different proteins exhibit significant variations in their overall hydrophilicity and hydrophobicity, since they carry varying amount of hydrophilic and hydrophobic amino acids. Serum and plasma contain a variety of proteins that exhibit hydrophilicity at different strength and may potentially interfere with an immunoreaction-based detection reagent. Moreover, variations in protein levels in individuals can result in different severity of interference with immunoreaction.

Currently, conventional methods for evaluating interference resistance, such as common endogenous and exogenous substances, fail to effectively reflect the interference resistance of a reaction system against hydrophobic interferents.

### SUMMARY

Based on this, there is a need to provide a method for preparing a hydrophobic interferent and the hydrophobic interferent prepared thereby. The hydrophobic interferent can be used to evaluate the interference resistance against hydrophobic interaction in an immunoreaction.

Specific technical solution is as follows.

In a first aspect of the present application, a method for preparing a hydrophobic interferent is provided. The method includes adding a lysing agent into a whole blood sample to fully lyse blood cells in the whole blood sample and release contents in the cells, followed by centrifugation to collect a supernatant, and adding an oxidizing agent to the supernatant to oxidize the contents, thereby enhancing hydrophobicity, followed by centrifugation to collect a supernatant, thereby obtaining the hydrophobic interferent.

In an embodiment, a source of the whole blood sample includes human or animal peripheral blood.

In an embodiment, the lysing agent includes Triton X-100.

In an embodiment, the oxidizing agent includes one or more of H₂O₂, urea peroxide, and potassium permanganate.

In an embodiment, the oxidizing agent has a final concentration of 0.5% (v/v) to 8% (v/v) in a reaction system, and the lysing agent has a final concentration of 0.05% (v/v) to 4% (v/v) in a reaction system.

In a second aspect of the present application, a hydrophobic interferent prepared by any of the aforementioned methods is provided.

In a third aspect of the present application, a kit including the aforementioned hydrophobic interferent is provided.

In a fourth aspect of the present application, a method for evaluating interference resistance against hydrophobic interaction in an immunoreaction is provided. The method includes evaluating interference resistance against hydrophobic interaction in the immunoreaction using the hydrophobic interferent or the kit.

In an embodiment, the method includes:
serially diluting the hydrophobic interferent to prepare a plurality of hydrophobic interferent solutions at different concentrations;
extracting the hydrophobic interferent solutions at different concentrations using an extraction agent, respectively, wherein an organic phase serves as an interferent sample and an aqueous phase serves as a control sample;
adding the interferent sample and the control sample, both with an immunoassay reagent, into individual reaction vessels to measure luminescence values; and
analyzing the interference resistance of the reaction vessels and/or the immunoassay reagent against hydrophobic interaction in the immunoreaction based on the luminescence values of the interferent sample and the control sample.

Optionally, the immunoassay reagent includes a solid-phase coating and/or a label conjugate.

Further optionally, the label conjugate includes an antigen or antibody labeled with a label, and the solid-phase coating includes an antigen or antibody coated on a solid phase.

In an embodiment, the immunoassay reagent includes a label conjugate.

Optionally, the immunoassay reagent includes a solid-phase coating and a label conjugate, and the reaction vessels are made of a hydrophobic material.

In a fifth aspect of the present application, use of the hydrophobic interferent or the kit in detecting the interference resistance of at least one of an immunodiagnostic reagent, a reaction vessel, an antigen to be tested, and an antibody to be tested in an immunoreaction against the hydrophobic interaction is provided.

Compared with prior art, the present application has the following advantages.

The present application provides a method for preparing a hydrophobic interferent in an immunoreaction. The prepared hydrophobic interferent can be used to study the interference resistance of an immunodiagnostic reagent, such as immunodiagnostic reagents used in POCT, ELISA, CLIA, etc., against the hydrophobic interferent. Instead of studies based on amplification with a large number of clinical samples, the assay of interference resistance against the hydrophobic interferent in advance in product development and validation phases, significantly reduces both time cycle cost and expense cost. Furthermore, reagents can be improved by identifying and optimizing in advance for potential interferences, thereby preventing false positive or false positive results in clinical sample testing.

### BRIEF DESCRIPTION OF THE DRAWINGS

FIG. 1 is a schematic diagram showing false-positive result caused by binding of an antibody coated on a solid phase to an antibody label mediated by a hydrophobic interferent.
FIG. 2 is a schematic diagram showing false-positive result caused by binding of an antigen coated on a solid phase to an antigen label mediated by a hydrophobic interferent.

### DETAILED DESCRIPTION

The above objects, features and advantages of the present application will become more apparent by describing in detail embodiments thereof with reference to the accompanying drawings. The following description sets forth in details to provide a thorough understanding of the present application. However, the present application may be carried out in many other ways different from those described herein. Similar modifications may be made by those skilled in the art without departing from the spirit of the present application. Therefore, the present application is not limited to the specific embodiments disclosed below.

Unless otherwise defined, all technical and scientific terms used herein have the same meaning as commonly understood by those skilled in the art to which the present application pertains. The terms used in the specification of the present application are merely for the purpose of describing specific embodiments and are not intended to limit the present application.

### Definition

The terms "first aspect," "second aspect," "third aspect," "fourth aspect," and "fifth aspect" are used for descriptive purposes only and should not be construed as indicating or implying importance relative to each other or implicitly specifying the quantity of the indicated elements. Thus, elements defined with "first aspect," "second aspect," "third aspect," "fourth aspect," or "fifth aspect" may explicitly or implicitly include at least one such element. In the present application, the term "a plurality of" means at least two, such as two, three, etc., unless expressly specified otherwise.

The term "and/or" includes any and all combinations of one or more of the associated listed items.

The term "hydrophobic interaction" refers to the force between two and/or more hydrophobic substances that enables mutual binding between the substances.

The abbreviated term "CLIA" refers to chemiluminescence immunoassay, which is an immunological analysis by directly labeling an antigen or antibody with a chemiluminescent agent.

The abbreviated term "POCT" refers to point-of-care testing, which primarily refers to rapid testing and analysis technologies which is performed near the patient's bedside, and enables testing technologies that can be conducted at the bedside, wards, or in locations other than central laboratories and clinical laboratories. includes but not limited to immunoturbidimetry and immunochromatography.

The abbreviated term "ELISA" refers to enzyme-linked immunosorbent assay, which is a qualitative and quantitative testing method that involves binding of a soluble antigen or antibody to a solid-phase carrier, and conducting an immunoreaction by antigen-antibody specific binding.

In an embodiment of the present application, a method for preparing a hydrophobic interferent is provided, which can be used to study interference resistance of a diagnostic reagent such as an immunodiagnostic reagent used in immunochromatography, ELISA, CLIA, etc. against hydrophobicity, to prevent false-positive result during testing a large number of clinical sample. Specifically, the method comprises the following steps a and b.

Step a: adding a lysing agent into a whole blood sample to fully lyse blood cells in the whole blood sample and release contents in the cells, followed by centrifugation to collect a supernatant.

In a specific embodiment, the lysing agent is preferably Triton X-100, and may be other cell lysis buffers such as a radio immunoprecipitation assay buffer (RIPA).

In another specific embodiment, the lysing agent has a final concentration of 0.05% (v/v) to 4% (v/v) in the reaction system. Optionally, the final concentration of the lysing agent in the reaction system may be in a range consisting of any of the following values: 0.05%, 0.1%, 0.5%, 1%, 2%, 3%, or 4%, preferably 1%, which allows thorough cell lysis at an appropriate concentration.

Specifically, the lysing agent is added into the whole blood sample, thoroughly mixed and incubated with the blood cells in the whole blood sample, to ensure fully lysis of blood cells in the whole blood sample and release of contents in the cells.

Specifically, the mode of mixing has no restriction as long as the two sets of components are homogeneously mixed. Mixing upside-down is optional.

Specifically, the incubation is carried out at room temperature for 20 min to 40 min, preferably 30 min. The centrifugation is carried out at 2 to 8 °C under a centrifugal force of 5,000 x g for 10 min to 30 min, preferably 20 min.

Step b: adding an oxidizing agent to the supernatant to oxidize the contents, thereby enhancing hydrophobicity, followed by centrifugation to collect a supernatant, thereby obtaining the hydrophobic interferent.

In a specific embodiment, the oxidizing agent includes one or more selected from the group consisting of H₂O₂, urea peroxide, and potassium permanganate.

In a specific embodiment, the oxidizing agent has a final concentration of 0.5% (v/v) to 8% (v/v) in the reaction system. Optionally, the final concentration of the oxidizing agent in the reaction system may be 0.5%, 1%, 2%, 3%, 4%, 5%, 6%, 7%, or 8%. Within this range, the prepared hydrophobic interferent exhibits strong hydrophobicity, with 4% (v/v) being preferred for greater hydrophobicity.

Specifically, the oxidation is carried out at 37°C for 10 to 20 minutes. Centrifugation is carried out at 2 to 8 °C at a centrifugal force of 5,000 x g for 10 to 30 min, preferably 20 min.

In an embodiment of the present application, a hydrophobic interferent prepared by the foregoing method and a kit including the hydrophobic interferent are further provided.

In an embodiment of the present application, a method for evaluating interference resistance against hydrophobic interaction in an immunoreaction is provided. The method includes evaluating interference resistance against hydrophobic interaction in the immunoreaction using the hydrophobic interferent or the kit.

In a specific embodiment, the method includes the following steps a to c:

Step a: serially diluting the hydrophobic interferent to prepare a plurality of hydrophobic interferent solutions at different concentrations.

Specifically, the hydrophobic interferent is diluted in physiological saline at a dilution ratio which may be 1:2, 1:4, 1:8, 1:16, 1:32, 1:64, etc.

Step b: extracting the hydrophobic interferent solutions at different concentrations using an extraction agent, respectively. An organic phase serves as an interferent sample and an aqueous phase serves as a control sample

Specifically, a volume ratio of the extraction agent to the hydrophobic interferent may be 2:1, 3:1, etc. as long as it is sufficient to extract the hydrophobic interferent.

Optionally, the extraction agent includes an organic solvent, such as chloroform, diethyl ether, toluene, or the like.

Step c: adding the interferent sample and the control sample, both with an immunoassay reagent, into individual reaction vessels to measure luminescence values.

Step d: analyzing the interference resistance of the reaction vessels and/or the immunoassay reagent against hydrophobic interaction in an immunoreaction based on the luminescence values of the interferent sample and the control sample.

Optionally, the immunoassay reagent includes a solid-phase coating and/or a label conjugate.

Specifically, the label conjugate comprises an antigen label or an antibody label. Further, the label includes, but is not limited to, acridinium ester, tris(bipyridine)ruthenium, biotin, etc.

Specifically, the solid-phase coating comprises a solid-phase coating antigen or a solid-phase coating antibody. Further, the solid phase includes magnetic beads, cellulose, glass, silicone rubber, polyacrylamide, polystyrene, etc.

In a specific embodiment, the aforementioned hydrophobic interferent is used to evaluate the interference resistance of the reaction vessel against hydrophobic interaction in an immunoreaction. Specifically, the evaluation is carried out according to steps a to d as described above. The immunoassay reagent includes the label conjugate and further includes physiological saline, which is used as a substitute for the solid-phase coating. Optionally, the specific steps and parameters for detecting the interferent sample and the control sample using the immunoassay reagent and for measuring the luminescence values refer to instructions for the immunoassay reagent or the immunoassay kit.

In another specific embodiment, the aforementioned hydrophobic interferent is used to evaluate the interference resistance of the immunoassay reagent against hydrophobic interaction in an immunoreaction. Specifically, the evaluation is carried out according to steps a to d as described above. The immunoassay reagent includes the solid-phase coating and the label conjugate, and the interferent sample and the control sample are separately detected to measure their luminescence values. The reaction vessel used in the detection is made of a hydrophobic material, such as glass material. Other specific steps and parameters for detecting or measuring the luminescence values may refer to an instruction for the immunoassay reagent or immunoassay kit.

Use of the aforementioned hydrophobic interferent or kit for testing the interference resistance of at least one of an immunodiagnostic reagent, a reaction vessel, an antigen to be tested, and an antibody to be tested, against hydrophobic interaction. Optionally, the immunodiagnostic reagent includes, but is not limited to, immunodiagnostic reagents used in POCT, ELISA, CLIA, etc.

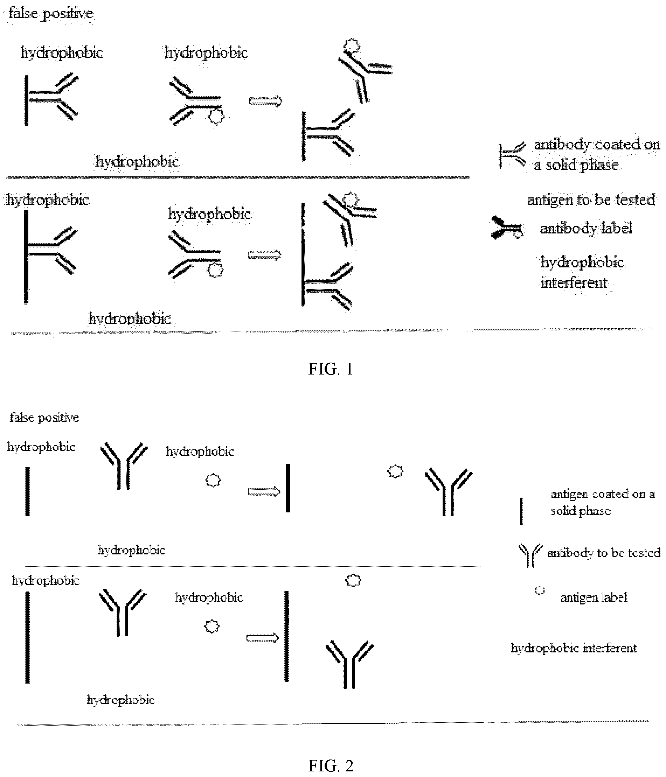

The hydrophobic interferent of the present application can directly mediate the bridging of a label conjugate to a reaction vessel made of a hydrophobic material, producing a false-positive signal. Additionally, the hydrophobic interferent can also bridge an antibody label or an antigen label to a solid-phase coating, producing a false-positive signal. Specifically, the antibody label may bind to a solid-phase coating antibody via the hydrophobic interferent prepared according to the present application, resulting in a false-positive signal (see Figure 1 for the reaction mechanism). The antigen label may bind to the solid-phase coating antigen via the hydrophobic interferent prepared according to the present application, resulting in a false-positive signal (see Figure 2 for the reaction mechanism).

### Detailed Examples

The embodiments of the present application will be described in detail below in connection with examples. It should be understood that these examples are only intended to illustrate the present application, instead of limiting the scope of the present application. For experimental methods where specific conditions are not indicated in the following examples, preferentially refer to the guidelines provided in the present application, or may follow laboratory manuals or conventional conditions in the field, conditions recommended by the manufacturer, or well-known experimental methods in the field.

In the specific examples described below, the measurement parameters of the raw material components may exhibit minor deviations within the weighing accuracy range, unless otherwise specified. For temperature and time parameters, acceptable deviations attributable to instrument measurement accuracy or operational precision are permitted.

Triton X-100 and H₂O₂ used in the following examples were purchased from Aladdin.

### Example 1

### Method for Preparing Hydrophobic Interferent

The interferent was prepared by processing a fully coagulated whole blood sample in a red-top blood collection tube. Triton X-100 was added to the blood collection tube at a final concentration of 1% (v/v). The tube was then capped and inverted repeatedly to ensure full and homogeneous mixing of Triton X-100 with blood cells, followed by incubation at room temperature for 30 minutes. The mixture was centrifuged at a centrifugal force of 5,000 x g and 2 to 8°C for 20 minutes, and the supernatant was collected. The supernatant was added with H₂O₂ at a final concentration of 4% (v/v), and incubated at 37 °C for 20 minutes. The oxidized supernatant was centrifuged at a centrifugal force of 5,000 x g and 2 to 8°C for 20 minutes. The resulting supernatant was collected as the interferent of the present application.

The term "final concentration" refers to the concentration percentage of either the lysing reagent or oxidizing agent relative to the reaction system.

### Example 2

The hydrophobic interferent prepared in example 1 was used to evaluate the interference resistance of a Type IV collagen (Col IV) chemiluminescence detection kit.

The specific procedure of the detection method was performed as follows:
1. Solution A containing 50 mM PB, 150 mM NaCl, 0.5% BSA, 0.05% Tween-20, and 0.1% ProClin300 (pH 7.0) was prepared.
2. Solution B containing 50 mM PB, 150 mM NaCl, 0.5% BSA, 2% Tween-20, and 0.1% ProClin300 (pH 7.0) was prepared.
3. Acridinium ester was labeled onto the Col IV antibody to prepare the antibody-label conjugate.
4. The antibody-label conjugate was diluted in the solution A to prepare an acridinium working solution A, and the antibody-label conjugate was diluted in the solution B to prepare an acridinium working solution B.
5. Magnetic bead working solution was replaced with physiological saline. Reagent A was prepared by combining the acridinium working solution A with physiological saline, while reagent B was prepared by combining the acridinium working solution B with physiological saline.
6. The interferent was diluted in physiological saline at ratios of 1:2, 1:4, 1:8, 1:16, 1:32, 1:64, and 1:128 to prepare interferents at different gradients. Physiological saline was used as control sample 1.
7. Chloroform was mixed with the interferent at a ratio of 2:1 by volume and rolled on a horizontal roller for 30 min, followed by centrifugation at a room temperature and at a centrifugal force of 5,000 x g for 15 min. The upper phase solution was transferred into an EP tube. Based on the "like dissolves like" principle, the strongly hydrophobic interferent was extracted with chloroform solution. The lower phase was chloroform and the extracted interferent, while the upper phase was an interferent-free solution. The upper phase was used as a control sample 2.
8. Operation was manually carried out with reference to the response parameters for the Col IV chemiluminescence detection kit (Cat. No. C86047, SHENZHEN YHLO BIOTECH CO., LTD). Reagent A, interferent samples at different gradients prepared at 1:2, 1:4, 1:8, 1:16, 1:32, and 1:64 dilution, control sample 1, and control sample 2 were added into reaction cups of PP (polypropylene) material respectively. The reaction cups were incubated for 10 min, followed by magnetic separation. A trigger solution and a pretrigger solution were added to detect the luminescence values. Reagent B, interferent samples at different gradients prepared at 1:2, 1:4, 1:8, 1:16, 1:32, and 1:64 dilution, control sample 1, and control sample 2 were added into reaction cups of PP (polypropylene) material respectively. The reaction cups were incubated for 10 min, followed by magnetic separation. A trigger solution and a pretrigger solution were added to detect the luminescence values.
9. Operation was manually carried out with reference to the response parameters for the Col IV chemiluminescence detection kit. Reagent A, interferent samples at different gradients prepared at 1:2, 1:4, 1:8, 1:16, 1:32, and 1:64 dilution, control sample 1, and control sample 2 were added into reaction cups of glass material respectively. The reaction cups were incubated for 10 min, followed by magnetic separation. A trigger solution and a pretrigger solution were added to detect the luminescence values. Reagent B, interferent samples at different gradients prepared at 1:2, 1:4, 1:8, 1:16, 1:32, and 1:64 dilution, control sample 1, and control sample 2 were added into reaction cups of glass material respectively. The reaction cups were incubated for 10 min, followed by magnetic separation. A trigger solution and a pretrigger solution were added to detect the luminescence values.

### Experimental Results:

The comparison of results by monitoring the reactions of the interferent sample and the control sample 2 with reagent A in the reaction cups of PP material shows a strong signal for the interferent sample, while almost no signal for the control sample 2. Control sample 2 was prepared by extracting the interferent sample with chloroform and removing the interferent therein. The fact that the interferent could be extracted with chloroform indicates that it is highly hydrophobic.

Since PP material is hydrophobic while glass material is highly hydrophilic, the hydrophobic interferent can bind to the reaction cups of PP material but not to the interferents of glass material. In this example, data obtained by detecting the interferent sample, control sample 1, and control sample 2 in reaction cups of PP material and of glass material with the reagent A was compared. The interferent could directly mediate the bridging of acridinium conjugate to the reaction cups of PP material, leading to false-positive results, whereas the results from the reaction cups of glass material were normal.

PP material is the most commonly used material for reaction cups in fully automated immunoassay instruments in the medical device industry. Instruments such as Architect and Alinity series from Abbott, as well as DXI series from Beckman, are all equipped with the reaction cups of PP material, which potentially brings a risk of false-positive results due to the binding of a label to the reaction cups mediated by the hydrophobic interferent in samples.

Tween-20 is one of the most commonly added surfactants in immunoassay reagents, and can increase the surface tension of a solution and reduce hydrophobic adsorption. The difference between reagent A and reagent B lies in their Tween-20 concentrations: the concentration of Tween-20 in reagent A is 0.05%, which is a common working concentration in commercial immunoassay reagents, while the concentration of Tween-20 in reagent B is 2%, the highest working concentration used in the immunoassay reagents. For the reagents A and B reacted in reaction cups of PP material, reagent B showed significantly lower signals than reagent A, but still failed to completely eliminate hydrophobic interference. Therefore, false-positive results caused by interference with hydrophobic interferent in samples cannot be completely improved by adding common Tween-20 into an immunoassay reagent.

The above describes an example where the acridinium-labeled anti-Col IV antibody binds to the reaction cups of PP material via the hydrophobic interferent. Further research program has shown that an acridinium-labeled antibody and an acridinium-labeled antigen in a plurality of different diagnostic kits can bind to the reaction cups via the hydrophobic interferent prepared according to the present application, leading to false-positive signals. The hydrophobic interferent prepared according to the present application binds to not only acridinium ester as a label, but also a plurality of antibodies and antigens. For the clinical samples, the interference manifestations in samples having high level of IgM and cardiovascular disease samples are consistent with those induced by the hydrophobic interferent of the present application.

**Table 1**

| Sample No. | Reaction cup of PP material | | Reaction cup of glass material | |
|---|---|---|---|---|
| | Reagent A | Reagent B | Reagent A | Reagent B |
| 1: 2 | 3159351 | 162853 | 318 | 428 |
| 1: 4 | 1048356 | 62481 | 388 | 351 |
| 1: 8 | 346527 | 19534 | 417 | 337 |
| 1: 16 | 125943 | 5167 | 409 | 418 |
| 1: 32 | 35942 | 1393 | 445 | 297 |
| 1: 64 | 98124 | 557 | 286 | 330 |
| Control sample 1 | 329 | 452 | 261 | 329 |
| Control sample 2 | 1051 | 394 | 359 | 423 |

### Example 3

The hydrophobic interferent prepared in example 1 was used to evaluate the interference resistance of an anti-TP (anti-treponema pallidum antibody) chemiluminescence detection kit.

The specific procedure of the detection method was performed as follows:
1. Solution A containing 50 mM PB, 150 mM NaCl, 0.5% BSA, 0.05% Tween-20, and 0.1% ProClin300 (pH 7.0) was prepared.
2. Solution B containing 50 mM PB, 150 mM NaCl, 0.5% BSA, 2% Tween-20, and 0.1% ProClin300 (pH 7.0) was prepared.
3. The purified treponema pallidum antigen was coated onto superparamagnetic microparticles to prepare a magnetic-bead coating. The purified treponema pallidum antigen was labeled with acridinium ester to prepare the label conjugate.
4. The magnetic-bead coating was diluted in solution A to prepare a magnetic-bead working solution and the acridinium label was diluted in solution A to prepare an acridinium working solution. Reagent A was prepared by combining the magnetic-bead working solution and the acridinium working solution.
5. The magnetic-bead coating was diluted in solution B to prepare a magnetic-bead working solution and the acridinium label was diluted in solution B to prepare an acridinium working solution. Reagent B was prepared by combining the magnetic-bead working solution and the acridinium working solution.
6. The interferent was diluted in physiological saline at ratios of 1:2, 1:4, 1:8, 1:16, 1:32, 1:64, and 1:128 to prepare interferents at different gradients. Physiological saline was used as control sample 1.
7. Chloroform was mixed with the interferent at a ratio of 2:1 by volume and rolled on a horizontal roller for 30 min, followed by centrifugation at a room temperature and at a centrifugal force of 5,000 x g for 15 min. The upper phase solution was transferred into an EP tube. Based on the "like dissolves like" principle, the strongly hydrophobic interferent was extracted with chloroform solution. The lower phase was chloroform and the extracted interferent, while the upper phase was an interferent-free solution. The upper phase was used as a control sample 2.
8. Operation was manually carried out with reference to the response parameters for the Anti-TP (anti-treponema pallidum antibody) chemiluminescence detection kit (Cat. No. C88049, SHENZHEN YHLO BIOTECH CO., LTD). Reagent A was used to detect the interferent samples at different gradients prepared at 1:2, 1:4, 1:8, 1:16, 1:32, and 1:64 dilution, control sample 1, and control sample 2 in reaction cups of glass material, respectively. The reaction cups were incubated for 10 min, followed by magnetic separation. A trigger solution and a pretrigger solution were added to detect the luminescence values.
9. Operation was manually carried out with reference to the response parameters for the Anti-TP (anti-treponema pallidum antibody) chemiluminescence detection kit. Reagent B was used to detect the interferent samples at different gradients prepared at 1:2, 1:4, 1:8, 1:16, 1:32, 1:64 dilution, control sample 1, and control sample 2 in the reaction cups of glass material, respectively. The reaction cups were incubated for 10 min, followed by magnetic separation. A trigger solution and a pretrigger solution were added to detect the luminescence values.

### Experimental Result

Due to the relatively low concentration of Tween-20 in reagent A, the hydrophobic interferent could bridge the magnetic bead coating and acridinium label, generating a false-positive signal. The concentration of Tween-20 in Reagent B in the reaction system increased, which significantly reduced the false-positive signals. However, false-positive results caused by interference with hydrophobic interferent in samples cannot be completely improved.

Tween-20 is one of the most commonly added surfactants in immunoassay reagents, and can increase the surface tension of a solution and reduce hydrophobic adsorption. The difference between reagent A and reagent B lies in their Tween-20 concentrations: The concentration of Tween-20 in reagent A is 0.05%, which is a common working concentration in commercial immunoassay reagents, while the concentration of Tween-20 in reagent B is 2%, the highest working concentration used in the immunoassay reagents. All the reaction cups used in this experiment are of glass material, and exhibits strong hydrophilicity. Therefore, potential signals caused by hydrophobic interferents bridging the reaction cups and acridinium label can be precluded.

The above example demonstrates the false-positive signal is generated by binding the acridinium label to magnetic bead coating through the hydrophobic interferent in the Anti-TP program. The hydrophobicity/hydrophilicity of the surfaces of magnetic beads varies significantly across manufacturers and the type of magnetic beads. For instance, tosyl (p-toluenesulfonyl) magnetic beads react based on a principle of an initial physical adsorption followed by cross-linking, and the magnetic beads from various manufacturers typically exhibit ubiquitous pronounced surface hydrophobicity. Consequently, antigens or antibodies coated on such magnetic beads are more susceptible to nonspecific adsorption due to the hydrophobic interaction, leading to false-positive signals. Further investigation revealed that when the hydrophobic interferents prepared according to the present application was tested using reagents in different programs from Abbott and Roche, false-positive was found for some programs.

**Table 2**

| Sample No. | reaction cups of glass material | |
|---|---|---|
| | Reagent A | Reagent B |
| 1: 2 | 121843 | 24568 |
| 1: 4 | 56312 | 10281 |
| 1: 8 | 21530 | 4621 |
| 1: 16 | 8954 | 2017 |
| 1: 32 | 4452 | 897 |
| 1: 64 | 1531 | 562 |
| Control Sample 1 | 346 | 463 |
| Control Sample 2 | 450 | 418 |

The technical features of the above-mentioned embodiments can be combined arbitrarily. In order to make the description concise, not all possible combinations of the technical features are described in the embodiments. However, as long as there is no contradiction in the combination of these technical features, the combinations should be considered as in the scope of the present application.

The above-described embodiments are only several implementations of the present specification, and the descriptions are relatively specific and detailed, but they should not be construed as limiting the scope of the present specification. It should be understood by those of ordinary skill in the art that various modifications and improvements can be made without departing from the concept of the present specification, and all fall within the protection scope of the present specification. Therefore, the patent protection of the present disclosure shall be defined by the appended claims and the description and the appended drawings may be used to interpret the content of the claims.

## Claims

1. A method for preparing a hydrophobic interferent comprising:
adding a lysing agent into a whole blood sample to fully lyse blood cells in the whole blood sample and release contents in the cells, followed by centrifugation to collect a supernatant; and
adding an oxidizing agent to the supernatant to oxidize the contents, thereby enhancing hydrophobicity, followed by centrifugation to collect a supernatant, thereby obtaining the hydrophobic interferent.

2. The method of claim 1, **characterized in that**, a source of the whole blood sample comprises human or animal peripheral blood.

3. The method of claim 1, **characterized in that**, the lysing agent comprises Triton X-100.

4. The method of claim 1, **characterized in that**, the oxidizing agent comprises one or more of H₂O₂, urea peroxide, and potassium permanganate.

5. The method of any one of claims 1 to 4, **characterized in that**, the oxidizing agent has a final concentration of 0.5% (v/v) to 8% (v/v) in a reaction system, and the lysing agent has a final concentration of 0.05% (v/v) to 4% (v/v) in the reaction system.

6. A hydrophobic interferent prepared by the method of any one of claims 1 to 5.

7. A kit comprising the hydrophobic interferent of claim 6.

8. A method for evaluating interference resistance against hydrophobic interaction in an immunoreaction comprising evaluating interference resistance against hydrophobic interaction in the immunoreaction using the hydrophobic interferent of claim 6 or the kit of claim 7.

9. The method of claim 8, **characterized in that**, the method comprises serially diluting the hydrophobic interferent to prepare a plurality of hydrophobic interferent solutions at different concentrations;
extracting the hydrophobic interferent solutions at different concentrations using an extraction agent, respectively, wherein an organic phase serves as an interferent sample and an aqueous phase serves as a control sample;
adding the interferent sample and the control sample, both with an immunoassay reagent, into individual reaction vessels to measure luminescence values; and
analyzing the interference resistance of the reaction vessels and/or the immunoassay reagent against hydrophobic interaction in the immunoreaction based on the luminescence values of the interferent sample and the control sample;
optionally, the immunoassay reagent comprises a solid-phase coating and/or a label conjugate. further optionally, the label conjugate comprises an antigen or antibody labeled with a label, and the solid-phase coating comprises an antigen or antibody coated on a solid phase.

10. The method of claim 9, **characterized in that**, the immunoassay reagent comprises a label conjugate;
optionally, the immunoassay reagent comprises a solid-phase coating and a label conjugate, and the reaction vessels are made of a hydrophobic material.

11. Use of the hydrophobic interferent of claim 6 or the kit of claim 7 in detecting interference resistance of at least one of an immunodiagnostic reagent, a reaction vessel, an antigen to be tested, and an antibody to be tested in an immunoreaction against hydrophobic interaction.
